# EUROPEAN PATENT SPECIFICATION

(11) **EP 0 861 638 B1**
(45) Date of publication and mention of the grant of the patent: **14.09.2005**
(21) Application number: 98301345.9
(22) Date of filing: 24.02.1998
(51) Int. Cl.: A61F 2/06

(54) **Modular endoluminal stent-grafts**
Modulares endoluminales Stent-Gewebe
Stent-prothése endoluminale modulaire

(30) Priority: 27.02.1997 US 806739
(43) Date of publication of application: 02.09.1998
(73) Proprietor: CORVITA CORPORATION, Florida 33122 (US)
(72) Inventor: Pinchuk, Leonard, Miami, Florida 33176 (US); Dereume, Jean-Pierre, 1000 Brussels (BE)
(74) Representative: Davies, Gregory Mark

(56) References cited:
- EP-A- 0 740 928
- WO-A-95/09586
- DE-A- 4 418 336
- US-A- 5 064 435
- WILSON G J ET AL: "A SELF-EXPANDING BIFURCATED ENDOVASCULAR GRAFT FOR ABDOMINAL AORTIC ANEURYSM REPAIR" ASAIO JOURNAL, vol. 42, no. 5, September 1996, pages M386-M393, XP000683606

## Description

### BACKGROUND OF THE INVENTION

### 1. Field of the Invention

The invention relates to an implantable prosthesis. In particular, the invention relates to endoluminal grafts and stent-grafts which are deployed in a blood vessel which has a varying diameter. The invention is particularly suited for repairing the aortic artery and daughter arteries, although it is not limited thereto.

### 2. State of the Art

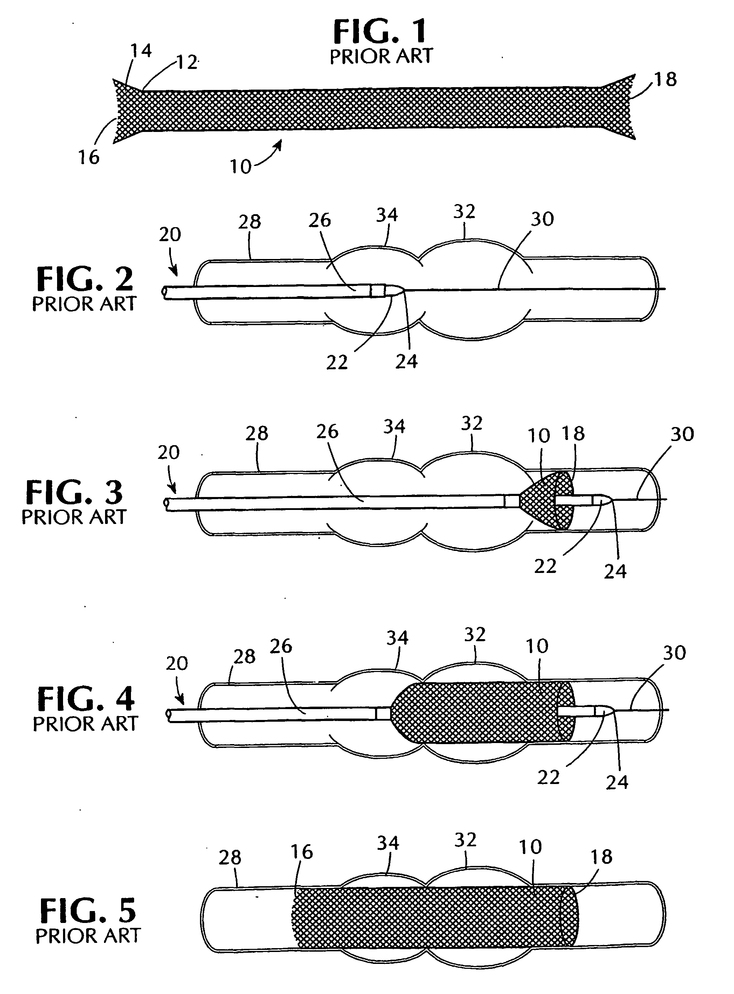

An endoluminal stent-graft typically includes tubular graft material which is affixed to the inside or outside of a woven metallic stent and is delivered to the damaged site of a blood vessel via a catheter. Endoluminal stent-grafts are most often used to repair blood vessels affected by a variety of lesions such as stenoses or aneurysms. A typical prior art stent, shown in Figures 1-6, is a metallic structure 10 made of braided wire 12 such as stainless steel, cobalt-chromium-nickel super alloys and combinations, co-extrusions or braised combinations of the above with tantalum, gold, platinum and the like. Stents are also made from memory alloys such as nitinol and the like. Typical stents are disclosed in US Patents Numbers 4,655,771 and 4,945,126 to Wallsten, and in UK Patent Number 1205743 to Didcott. Generally, the wires 12 are braided with a large pick size, i.e. with relatively large interstices 14 between the wires, so that axial expansion of the stent causes a diametrical compression of the stent. Most often the braiding and/or the metal chosen for the wires yields a resilient stent which is self-expanding. However, some stents are not self-expanding and are expanded with the use of a balloon catheter. In the case of self-expanding stents, the proximal and distal ends 16, 18 of the stent are usually flared when expanded. US5064435 describes a stent formed of two pieces having flared ends, where the flared ends are of substantially equal diameters.

While endoluminal stents have been used without any graft material when repairing stenoses, it is now generally preferred to use a graft material in combination with the stent when repairing stenoses as well as when repairing aneurysms. The graft material most often used in endoluminal grafts is a PET or polytetrafluroethylene (PTFE) material which is folded to reduce its size and which is attached to one or both ends of a radially expandable stent by means of sutures. When the stent self-expands or is balloon expanded, the graft unfolds around the stent. The above-referenced parent application discloses a stent-graft which incorporates an improved self-expanding graft material.

While the primary use of endoluminal stents is to treat stenoses, stents are also sometimes used in conjunction with graft material to bridge aneurysms. The advantage of using a stent in bridging aneurysms is that the expanded stent helps to fix the graft in place, can eliminate the need for sutures, and may provide some additional resistance to hoop stress. Prior art Figures 2-5 illustrate the deployment of a stent-graft to bridge an aneurysm.

Referring now to Figures 2-5, the ends of the stent 10 are axially displaced inside an introducer 20 which includes an inner catheter 22 having a soft (dilator) tip 24 and an outer sheath 26. The introducer 20 is delivered through a blood vessel 28 with the aid of a guide wire 30 which is inserted through the lumen of the inner catheter 22. The introducer 20 is guided over the guide wire 30 to the site of an aneurysm, in this case two adjacent aneurysms, namely distal aneurysm 32 and proximal aneurysm 34. With the aid of fluoroscopy, the introducer 20 is positioned so that the soft tip 24 is located distally relative to the distal aneurysm 32. The outer sheath 26 is drawn proximally while the inner catheter 22 is held stationary. This releases the distal end 18 of the stent 10 which self-expands to the inner diameter of the vessel 28 as shown in Figure 3. Continued proximal movement of the outer sheath 26 releases the remainder of the stent 10 as shown in Figure 4 until the proximal end 16 of the stent 10 expands to the inner diameter ofthe vessel 28 proximal of the proximal aneurysm 34 as shown in Figure 5, after which the introducer 20 and the guide wire 30 are removed from the vessel 28.

From the foregoing, it will be appreciated that by using an appropriately sized stent-graft, the aneurysms 32, 34 in Figures 2-5 are effectively bridged utilizing the procedure described above. In particular, the stent-graft must be long enough so that its proximal and distal ends extend beyond the aneurysms and expand into healthy areas of the blood vessel. Moreover, the stent-graft must be chosen to have the appropriate expanded diameter so that a good seal is made between the stent-graft and the inner wall of the blood vessel. However, the diameter should not be so large that when the stent expands, the outward pressure of the expanding stent damages the wall of the blood vessel.

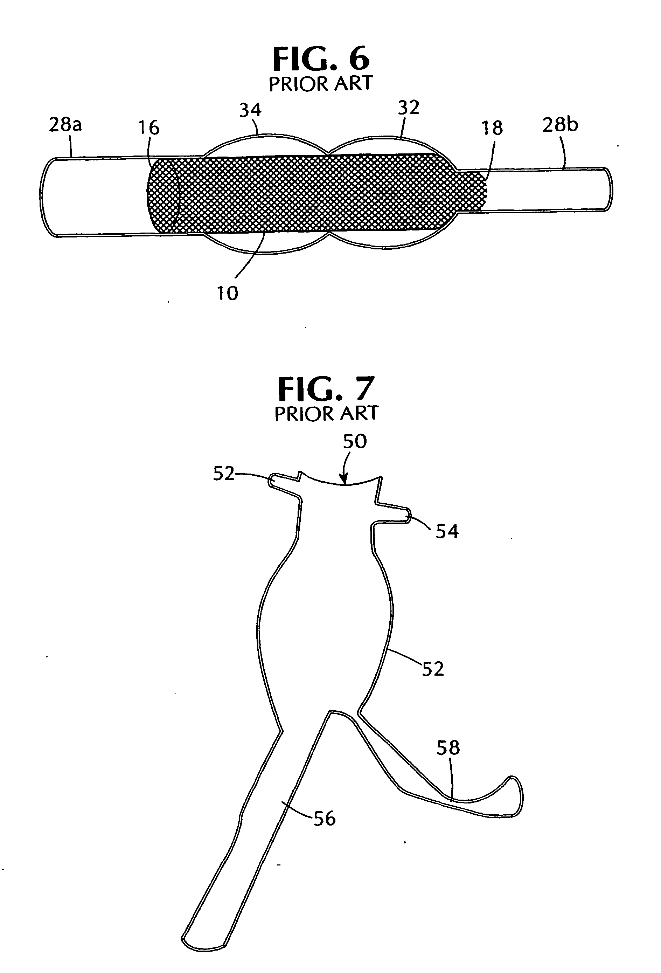

Because of the above considerations, it is difficult or impossible to bridge an aneurysm with a stent-graft when the diameter of the blood vessel on either side of the aneurysm differs by any significant amount. For example, as shown in Figure 6, the distal end 18 of a stent-graft 10 is greatly compressed as compared to the proximal end 16 when the stent-graft is used to bridge aneurysms 32, 34 where the diameter of the vessel 28 on the proximal side 28a of the aneurysms 32, 34 is substantially greater than the diameter of the vessel on the distal side 28b of the aneurysms 32, 34. Depending on the nature of the particular stent-graft, this can cause damage to the vessel on the distal side 28b or can result in an inward tapering of the distal end 18 of the graft to a "cigar shape". In the former situation, the damage can result in an additional aneurysm or rupture of the vessel. In the latter situation, the distal end 18 of the graft can obstruct the flow of blood, or jeopardize the seal between the distal end 18 and the inner wall of the vessel 28b. In the case of obstruction, occlusion of the vessel may occur which can be catastrophic to the patient. In the case of seal weakening, blood will enter into the aneurysmal sac and promote continued growth of the aneurysm.

More often than not the vessels of the vascular tree especially in the abdominal aortic artery exhibit the joining of vessels having very different diameters. For example, as shown in Figure 7, the abdominal aortic artery 50 is the trunk from which the renal arteries, right 52, left 54 and the iliac arteries, right 56, left 58 proceed. An aortic aneurysm 60 between the renal arteries and the iliac arteries is very difficult to bridge since the diameter of the aortic artery is approximately 25mm, whereas the diameter of the iliac artery is about 12mm. A stent-graft having a diameter of 27mm will fit well in the aortic artery, but will be too large for the iliac artery. A 13mm diameter stent-graft will fit well in the iliac artery, but will be too small for the aortic artery.

The above-referenced parent application discloses a bifurcated stent-graft which is useful in repairing an abdominal aortic aneurysm and iliac aneurysm. The bifurcated graft is located in the abdominal aortic artery just above the iliac arteries with its bifurcated end closest to the iliac arteries. The bifurcated stent-graft effectively bypasses an aneurysm in the aortic artery and provides a radiopaque bifurcated guide to the iliac arteries. Once the bifurcated graft is deployed, an additional graft may be deployed in each of the iliac arteries. The additional grafts are deployed through the legs of the bifurcated stent-graft. The bifurcated legs provide separate fluid couplings for the two additional grafts so that blood can flow from the aortic artery to both iliac arteries.

Subsequent to the development of the bifurcated stent-graft of the parent application, additional discoveries have been made regarding the use of multiple stent-grafts to bridge vessels of different diameter. In particular, it is sometimes desirable to bridge the aortic artery with only one of the iliac arteries.

In addition, it has been discovered that in some situations where a stent-graft has been implanted to bridge an aneurysm, the stent-graft will continue to expand radially long after the time of implantation. This is particularly likely where there is continuous progression of aneurysmal disease and dilation ofthe neck of the aneurysm-. The continued radial expansion of the stent-graft results in a continued axial shortening of the stent-graft which often results in the ends of the stent-graft becoming dislodged from the blood vessel whereupon the prosthesis floats free inside the aneurysm causing serious danger to the patient.

### SUMMARY OF THE INVENTION

It is therefore an object of the invention to provide endoluminal stent-grafts which are useful for bridging vessels of different diameter.

It is still another object of the invention to provide an endoluminal stent-graft with a limited radial expandability and limited axial compressibility.

In accord with these objects which will be discussed in detail below, the modular endoluminal stent-grafts of the present invention include at least two different sized stent-grafts which are deployed one within the other. According to one embodiment of the invention, a first stent-graft is provided having a flared end which is expandable to a first diameter and a midsection which is expandable to a second diameter smaller than the first diameter. A second stent-graft is also provided having an end which is expandable to a diameter which engages the midsection ofthe first stent-graft. The first embodiment of the invention is deployed by expanding the first stent-graft such that its flared end engages a large diameter vessel, then expanding the second stent-graft inside the midsection ofthe first stent graft and inside a small diameter vessel such that the second stent graft engages the small diameter vessel and the midsection of the first stent-graft. Both the first and second stent-grafts may be manufactured in a conventional manner using conventional materials. According to a second embodiment of the invention, the midsection of the first stent-graft is reinforced with a flexible member to restrict the midsection from ballooning due to the outward pressure of the second stent-graft deployed within the lumen of the first stentgraft. The reinforcing member may be applied to all or a portion of the stent-graft. The reinforcing member is also useful in preventing the stent-graft from ballooning due to the presence of static blood pressure over time after implantation.

According to other aspects of the invention, the first stent-graft is provided with two flared ends and the second stent graft is provided with or without flared ends.

According to still another embodiment of the invention, three or more stent-grafts of different expanded diameter are deployed one within the other.

According to another embodiment of the invention, two or more stent-grafts of different diameter are pre-coupled to each other prior to deployment and are deployed using a single introducer in substantially one step.

According to still other aspects of the invention, the second and/or third stent-grafts are reinforced with a flexible member to restrict the midsection from ballooning.

Additional objects and advantages of the invention will become apparent to those skilled in the art upon reference to the detailed description taken in conjunction with the provided figures.

### BRIEF DESCRIPTION OF THE DRAWINGS

Figure 1 is a side elevation view of a prior art stent;
Figure 2 is a broken side elevation view in partial section of a prior art stent introducer during a first stage of deployment in a blood vessel with two adjacent aneurysms;
Figures 3-5 are views similar to Figure 2 showing the subsequent stages of deployment according to the prior art;
Figure 6 is a view similar to Figure 5 showing a blood vessel which has different diameters on either side of the aneurysms;
Figure 7 is a schematic view of an abdominal aortic aneurysm;
Figure 8 is a side elevation view ofa first stent-graft in a modular system according to the invention;
Figure 9 is a schematic view ofthe stent-graft of Figure 8 deployed in an abdominal aortic aneurysm;
Figure 10 is a view similar to Figure 9 showing a second stent-graft in a modular system according to the invention deployed inside the first stent-graft and inside the right iliac artery;
Figure 11 is a side elevation view of a second embodiment of a first stent-graft according to the invention having a flexible reinforcement;
Figure 11a is a view similar to Figure 11 of a stent-graft according to the invention having another type of flexible reinforcement;
Figure 11b is a view similar to Figure 11a of a stent-graft according to the invention having still another type of flexible reinforcement;
Figure 12 is a schematic view of a modular stent-graft system according to the invention having three stent-grafts where the second is deployed inside the first and the third is deployed inside the second;
Figure 13 is a schematic view of a modular stent-graft system according to the invention in which stent-grafts of different diameter are pre-coupled to each other prior to deployment; and
Figure 14 is a reduced schematic view of the stent-graft system of Figure 13 in a "pulled-down" state.

### DETAILED DESCRIPTION OF THE PREFERRED EMBODIMENTS

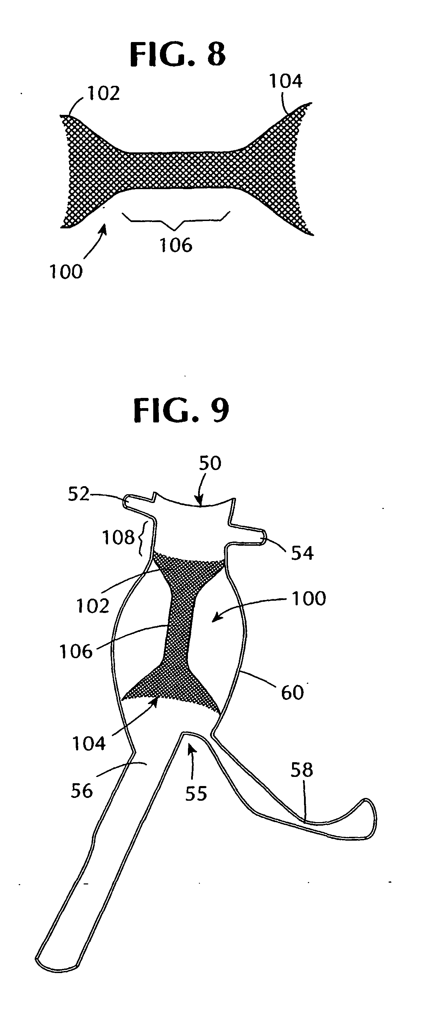

Referring now to Figures 8-10, a first stent-graft 100 in a modular system according to the invention has a flared proximal end 102, a flared distal end 104, and a midsection 106. The proximal end 102 is provided with an expanded diameter equal to or slightly larger than the inner diameter of the proximal end of an aneurysm that is to be bridged, e.g. the neck 108 of the abdominal aortic artery 50. The midsection 106 is provided with an expanded diameter equal to or slightly smaller than the inner diameter ofthe distal end 55 of an aneurysm that is to be bridged, e.g. the right iliac artery 56. The stent-graft 100 may be manufactured according to conventional methods with conventional materials, but is preferably manufactured using the methods and materials described in the above-referenced parent application. A second stent-graft 200 in a modular system according to the invention has a proximal end 202, a distal end 204, and a midsection 206. The expanded diameter of the proximal end 202 is dimensioned to engage the expanded interior of the midsection 106 of the first stent-graft 100 and the expanded diameter of the distal end 204 is dimensioned to engage the interior of the distal end of an aneurysm that is to be bridged, e.g. the right iliac artery 56. The stent-graft 200 may be manufactured according to conventional methods with conventional materials, but is preferably manufactured using the methods and materials described in the above-referenced parent application.

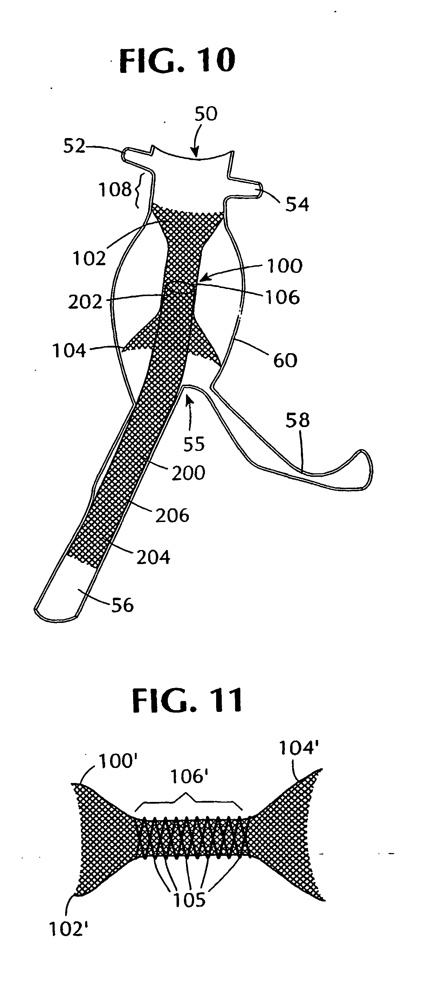

The modular stent-grafts 100 and 200 are deployed in the following manner which is illustrated by way of example in Figures 9 and 10 which depict deployment in an abdominal aortic aneurysm. The first stent-graft 100 is compressed into an introducer (not shown) and delivered to the a point distal ofthe renal arteries 52, 54 using conventional methods (see Figures 2-5). The stent-graft 100 is deployed such that the proximal end 102 of the stent-graft 100 expands into the neck 108 ofthe aortic artery distal of the renal arteries 52, 54 but proximal of the aortic aneurysm 60. The expanded distal end 104 rests in the aneurysm itself and serves to stabilize the position of the midsection 106 as shown in Figure 9. The introducer (not shown) is withdrawn and the second stent-graft 200 is compressed into the same or another introducer and delivered through the first stent-graft 100 to a point within the right iliac artery 56. The second stent-graft 200 is deployed such that the proximal end 202 of the second stent-graft expands into the midsection 106 of the first stent-graft 100 and the distal end 204 of the second stent-graft expands into the right iliac artery.

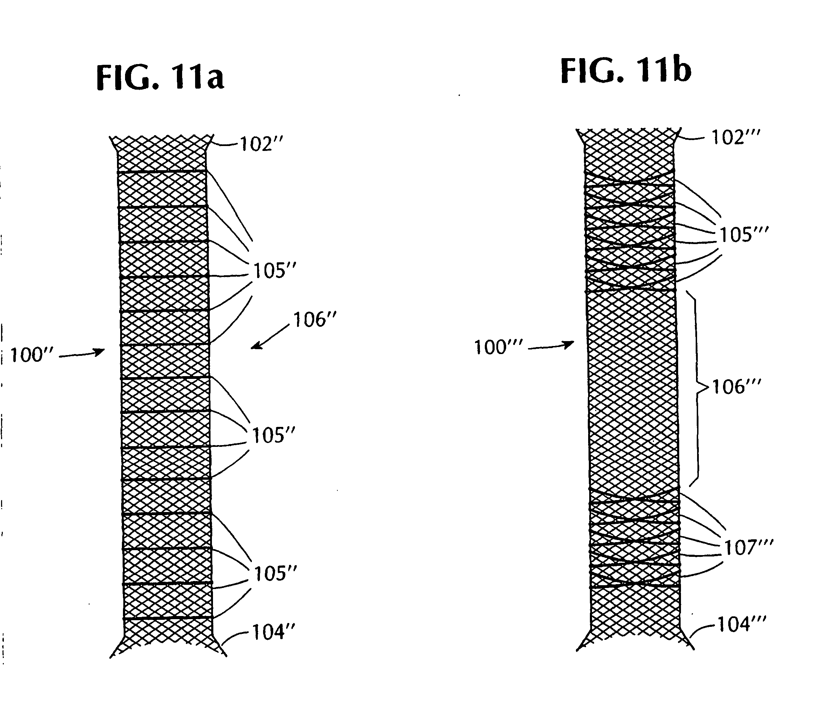

As mentioned above, both the first and second stent-grafts may be manufacturedaccording to conventional methods with conventional materials or using the methods and materials described in the above-referenced parent application. In addition, the second stent-graft may be made with fewer wires and/or with smaller wires in order that it fit properly in the iliac artery. The first stent-graft 100 may also be provided with midsection reinforcement as shown in Figure 11.

Turning now to Figure 11, there is illustrated a stent-graft 100' which is similar to the first stent-graft 100 described above. The stent-graft 100' has a flared proximal end 102', a flared distal end 104', and a midsection 106'. According to this embodiment, the stent-graft 100' has a flexible reinforcement 105' attached to the midsection 106' which restricts the midsection from ballooning when another (second) stent-graft is expanded inside the midsection. The flexible reinforcement 105' may be formed from sutures, knits, weaves, braids, wires, or another stent. The reinforcement 105' may be attached to the inside or the outside of the midsection. Suitable materials for the reinforcement 105' include polyethylene teraphthalate, nylon, polytetrafluoroethylene, polyolefin, polyamide, polycarbonate, polycarbonate urethane, metallic wire such as tantalum, stainless steel, titanium, annealed cobalt-chromium-nickel, etc. The reinforcement may be attached to the stent by suturing, gluing, hooks, welds or any other method which does not interfere with the compression of the stent. As shown in Figure 11, the reinforcement 105' is a substantially continuous member or members. In addition, such a reinforcement may be applied to all or part ofthe second stent-graft 200 described above in order to prevent ballooning of the second stent-graft in regions of high blood pressure, or in instances where the stent-graft is compressed axially during deployment.

Turning now to Figure 11a, there is shown a stent-graft 100" which is similar to the first stent-graft 100 described above. The stent-graft 100" has a flared proximal end 102", a flared distal end 104", and a midsection 106". According to this embodiment, the stent-graft 100" has a flexible reinforcement 105" attached to the midsection 106" and extending along substantially its entire length. In addition, in this embodiment, the reinforcement is formed from a series of discrete members which are axially spaced apart from each other. An advantage of using discrete members is that the stent-graft can be trimmed on the operating table without risking detachment of the ends of the reinforcement.

This advantage can also be achieved with a reinforcement which is inlay knitted or woven into the graft component of the stent-graft, a reinforcement which is added to the outside of the stent-graft, or a reinforcement which is located between the stent and the graft.

Figure 11b shows a stent-graft 100"' which is similar to the first stent-graft 100 described above. The stent-graft 100"' has a flared proximal end 102"', a flared distal end 104''', and a midsection 106"'. According to this embodiment, the stent-graft 100"' has a first flexible reinforcement 105''' located between the proximal end 102''' and the midsection 106''' and a second flexible reinforcement 107''' located between the distal end 104''' and the midsection 106"'. An advantage of this configuration is that it allows a small amount of additional axial compressibility which can be helpful during deployment. For example, if the stent is too long, it can be compressed axially to fit in the desired space. In addition, the pitch angle of the reinforcements 105''', 107''' can be made lower to add a small amount of longitudinal compressibility to the stent-graft while still maintaining a restriction on the radial expandability of the stent-graft.

Common to all of the embodiments of the reinforced stent-graft is the feature that the reinforcement is flexible enough to allow the stent-graft to be pulled down to a small diameter for delivery to the deployment site, but be strong enough to limit the radial expansion of the stent-graft beyond a diameter which is substantially equal to the resting diameter of the stent-graft.

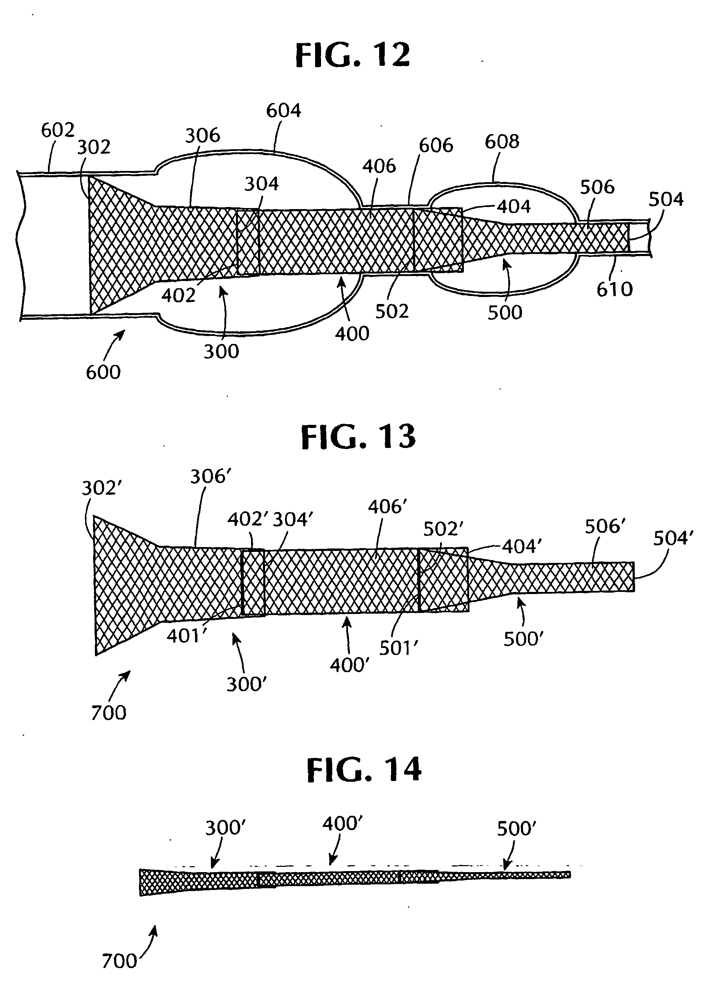

The modular stent-graft system of the invention may include more than two stent-grafts. For example, as shown in Figure 12, a modular system may include three stent-grafts 300, 400, 500 for bridging two aneurysms 604, 608 in a blood vessel 600 which exhibits three different diameters 602, 606, 610. As shown in Figure 12, the first stent-graft 300 has a flared proximal end 302, a non-flared distal end 304, and a midsection 306. The second stent-graft 400 has a non-flared proximal end 402, a non-flared distal end 404, and a midsection 406. The third stent-graft 500 has a flared proximal end 502, a non-flared distal end 504, and a midsection 506. The flared proximal end 302 ofthe first stent-graft 300 has an expanded diameter which fits securely in the large diameter portion 602 of the blood vessel 600 proximal of the first aneurysm 604. The second stent-graft 400 has a substantially constant expanded diameter which causes its proximal end 402 to fit securely in the midsection 306 of the first stent-graft 300 and its midsection 406 to fit securely in the smaller diameter section 606 ofthe blood vessel 600 between the first aneurysm 604 and the second aneurysm 608. The flared proximal end 502 of the third stent-graft 500 has an expanded diameter which fits securely in the midsection 406 of the second stent-graft 400; and the non-flared distal end 504 has an expanded diameter which fits securely in the smallest diameter portion 610 ofthe blood vessel 600 distal of the second aneurysm 608. The modular stent-grafts of Figure 12 are deployed in a manner similar to the stentgrafts shown in Figure 10, i.e. by deploying the proximal stent-graft first, and then following with distal stent-grafts. Although Figure 12 shows three stent-grafts with increasingly smaller diameters, the proximal to distal diameter change need not be from larger to smaller. For example, if the aneurysm 608 were located proximal of the aneurysm 604, the stent-grafts could be deployed in a different order or in the same order but with their proximal and distal ends reversed. That is, the stent-graft 400 could be deployed first and the stent-grafts 300 and 500 could be deployed inside the stent-graft 400. In this situation, it would be advantageous for the entire length of the stent-graft 400 to be reinforced. Alternatively, the stent-graft 300 could be deployed first with its end 302 being deployed distally, etc.

Referring now to Figures 13 and 14, a modular stent-graft system 700 is shown. The stent-graft system 700 has three stent-grafts 300', 400', and 500' which are similar to the stent-grafts 300, 400, and 500 described above. As shown in Figure 13, the primed reference numerals (e.g. 302') refer to features of the stent-grafts 300', 400', and 500' which are similar to features of the stent-grafts 300, 400, and 500 described above. According to this embodiment of the invention, the proximal end 402' of the stent-graft 400' is pre-coupled to the midsection 306' of the stent-graft 300' and the proximal end 502' of the stent-graft 500' is pre-coupled to the midsection 406' of the stent-graft 400'. The pre-coupling may be effected at the time of manufacture, or by a practitioner prior to deployment of the modular stent-graft system. As shown in Figure 13, the pre-coupling is accomplished with sutures 401' and 501'. However, the stent-grafts may also be coupled to each other by wires, adhesives, welds, or by using any other suitable coupling method. After the stent-grafts 300', 400', and 500' are coupled to each other, they are "pulled down" as a single unit with the aid of an introducer to a compressed state as shown in Figure 14 for deployment.

There have been described and illustrated herein several embodiments of modular endoluminal stent-grafts and methods for their use. While particular embodiments of the invention have been described, it is not intended that the invention be limited thereto, as it is intended that the invention be as broad in scope as the art will allow and that the specification be read likewise. Thus, while particular dimensions and materials have been disclosed, it will be appreciated that other dimensions and materials could be utilized. Also, while the stent-grafts have been shown for use in bridging aneurysms, it will be recognized that the modular system of stent-grafts could be used to bridge other types of lesions. Moreover, while particular configurations have been disclosed in reference to flared ends and reinforcing members, it will be appreciated that other configurations could be used as well. For example, the modular stent-graft 100 described with reference to Figure 10 could be provided with a single flared end, the proximal end, rather than two flared ends, in order to fit in certain tortuous arteries. Also, it is possible to utilize a bifurcated stent (as shown in the parent application) as a component in a modular stent system and use an occluding device to block one of its legs.

It will therefore be appreciated by those skilled in the art that yet other modifications could be made to the provided invention without deviating from its scope as so claimed.

## Claims

1. A modular stent-graft system for bridging a lesion in a blood vessel having a relatively large diameter on the proximal side of the lesion, a relatively small diameter on the distal side of the lesion, and an intermediate region between said proximal side and said distal side having a diameter larger than said relatively large diameter, said system comprising:
a stent-graft (100) having a first end (104), a second end (102) and a midsection (106), said first end (104) of said first stent-graft having an expanded diameter equal to or slightly larger than the intermediate region diameter of the blood vessel, said midsection (106) of said stent-graft (100) having an expanded diameter which is approximately equal to said relatively small diameter of said blood vessel,
wherein said second end (102) of said first stent-graft (100) has an expanded diameter which is equal to or slightly larger than the relatively large diameter of the blood vessel, such that when said system is deployed, said first end (104) of said stent-graft (100) can engage the intermediate region of the blood vessel, and said second end (102) of said first stent-graft (100) can engage the proximal side of the lesion in the blood vessel, and
a second stent-graft (200) having a first end (202) and a second end (204), said first end (202) of said second stent-graft (200) having an expanded diameter equal to or slightly larger than said expanded diameter of said midsection (106) of said first stent-graft (100) and said second end (204) of said second stent-graft (200) having an expanded diameter which is equal to or slightly larger than the relatively small diameter of the blood vessel, such that when said system is deployed, said first end (202) of said second stent-graft (200) can engage said midsection (106) of said first stent-graft (100), and said second end (204) of said second stent-graft (200) can engage the blood vessel on the distal side of the lesion.

2. A system according to claim 1, wherein said first end (104) and/or second end (102) of said stent-graft (100) is flared.

3. A system according to claim 1 or claim 2, further comprising:
a third stent-graft (500) having a first end (502) and a second end (504), said first end of said third stent-graft having an expanded diameter equal to or slightly larger than said expanded diameter of said midesection (206) of second stent-graft (200) such that when said system is deployed, said first end (502) of said third stent-graft (500) can engage said midsection (206) of said second stent-graft (200).

4. A system according to claim 3, wherein said first end (502) of said third stent-graft (500) is flared.

5. A system according to any of claims 1 to 3, wherein said first end (104) and/or said second end (102) of said first stent-graft (100) is flared.

6. A system according to any of claims 1 to 5, wherein at least said midsection (106) of said first stent-graft (100) is reinforced with a flexible material (105).

7. A system according to claim 6, wherein said flexible material (105) comprises polythylene terephthalate, nylon, polytetrafluoroethylene, polyolefin, polyamide, polycarbonate, polycarbonate urethane or metallic wire.

8. A system according to claim 6, wherein said flexible material (105) is selected from sutures, knits, weaves, braids, wires and stents.

9. A system according to any of claims 1 to 8, wherein said first end (202) of said second stent-graft (200) is coupled to said first stent-graft (100) by one of sutures, wires, adhesive and welds.

10. A modular stent-graft system according to any one of claims 1 to 9 wherein said midsection of said first stent-graft (100) defines a first lumen therethrough, and said midsection (206) of said second stent-graft (200) defines a second lumen therethrough, said first end (202) of said second stent- graft (200) being coupled to said first stent-graft (100) such that said first lumen is substantially contiguous with said second lumen.

11. A system according to claim 10, wherein said first end (202) of said second stent-graft (200) is coupled to said midsection (106) of said first stent-graft (100).

## Patentansprüche

1. Modulares Stent-Transplantatsystem zum Überbrücken einer Läsion in einem Blutgefäß mit einem relativ großen Durchmesser auf der proximalen Seite der Läsion, einem relativ kleinen Durchmesser auf der distalen Seite der Läsion und einem Zwischenbereich zwischen der proximalen Seite und der distalen Seite mit einem größeren Durchmesser als dem relativ großen Durchmesser, wobei das System umfasst:
ein Stent-Transplantat (100) mit einem ersten Ende (104), einem zweiten Ende (102) und
einem Mittelabschnitt (106), wobei das erste Ende (104) des ersten Stent-Transplantats einen expandierten Durchmesser gleich oder etwas größer als den Zwischenbereichsdurchmesser des Blutgefäßes aufweist, worin der Mittelabschnitt (106) des Stent-Transplantats (100) einen expandierten Durchmesser aufweist, der in etwa gleich dem relativ kleinen Durchmesser des Blutgefäßes ist,
worin das zweite Ende (102) des ersten Stent-Transplantats (100) einen expandierten Durchmesser aufweist, der gleich oder etwas größer als der relativ große Durchmesser des Blutgefäßes ist, so dass dann, wenn das System eingesetzt wird, das erste Ende (104) des Stent-Transplantats (100) in den Zwischenbereich des Blutgefäßes eingreifen kann und das zweite Ende (102) des ersten Stent-Transplantats (100) in der proximalen Seite der Läsion im Blutgefäß eingreifen kann, und
ein zweites Stent-Transplantat (200) mit einem ersten Ende (202) und einem zweiten Ende (204), worin das erste Ende (202) des zweiten Stent-Transplantats (200) einen expandierten Durchmesser gleich oder etwas größer als der expandierte Durchmesser des Mittelabschnitts (106) des ersten Stent-Transplantats (100) aufweist und das zweite Ende (204) des zweiten Stent-Transplaniats (200) einen expandierten Durchmesser aufweist, der gleich oder etwas größer als der relativ kleine Durchmesser des Blutgefäßes ist, so dass dann, wenn das System eingesetzt wird, das erste Ende (202) des zweiten Stent-Transplantats (200) in den Mittelabschnitt (106) des ersten Stent-Transplantats (100) eingreifen kann und das zweite Ende (204) des zweiten Stent-Transplantats (200) in das Blutgefäß auf der distalen Seite der Läsion eingreifen kann.

2. System nach Anspruch 1, worin das erste Ende (104) und/oder das zweite Ende (102) des Stent-Transplantats (100) ausgestellt sind.

3. System nach Anspruch 1 oder Anspruch 2, zusätzlich umfassend:
ein drittes Stent-Transplantat (500) mit einem ersten Ende (502) und einem zweiten Ende (504), worin das erste Ende des dritten Stent-Transplantats einen expandierten Durchmesser gleich oder etwas größer als der expandierte Durchmesser des Mittelabschnitts (206) des zweiten Stent-Transplantats (200) so aufweist, dass dann, wenn das System eingesetzt wird, um das erste Ende (502) des dritten Stent-Transplantats (500) den Mittelabschnitt (206) des zweiten Stent-Transplantats (200) eingreifen kann.

4. System nach Anspruch 3, worin das erste Ende (502) des dritten Stent-Transplantats (500) ausgestellt ist.

5. System nach einem der Ansprüche 1 bis 3, worin das erste Ende (104) und/oder das zweite Ende (102) des ersten Stent-Transplantats (100) ausgestellt sind.

6. System nach einem der Ansprüche 1 bis 5, worin mindestens der Mittelabschnitt (106) des ersten Stent-Transplantats (100) mit einem flexiblen Material (105) verstärkt ist.

7. System nach Anspruch 6, worin das flexible Material (105) Polyethylenterephthalat, Nylon, Polytetrafluorethylen, Polyolefin, Polyamid, Polycarbonat, Polycarbonaturethan oder Metalldraht umfasst.

8. System nach Anspruch 6, worin das flexible Material (105) ausgewählt ist unter Nähten, Strickverbindungen, Webverbindungen, Zöpfen, Drähten und Stents.

9. System nach einem der Ansprüche 1 bis 8, worin das erste Ende (202) des zweiten Stent-Transplantats (200) an das erste Stent-Transplantat (100) durch eines von Nähten, Drähten, Klebmaterial (Adhäsiv) und Schweißverbindungen gekoppelt ist.

10. Modulares Stent-Transplantatsystem nach einem der Ansprüche 1 bis 9, worin der Mittelabschnitt des ersten Stent-Transplantats (100) ein erstes Lumen durch dieses hindurch definiert und worin der Mittelabschnitt (206) des zweiten Stent-Transplantats (200) ein zweites Lumen durch dieses hindurch definiert, worin das erste Ende (202) des zweiten Stent-Transplantats (200) an das erste Stent-Transplantat (100) so gekoppelt ist, dass das erste Lumen im wesentlichen angrenzend an das zweite Lumen ist.

11. System nach Anspruch 10, worin das erste Ende (202) des zweiten Stent-Transplantats (200) an den Mittelabschnitt (106) des ersten Stent-Transplantats (100) gekoppelt ist.

## Revendications

1. Système modulaire de stent-prothèse pour ponter une lésion dans un vaisseau sanguin ayant un diamètre relativement grand sur le côté proximal de la lésion, un diamètre relativement petit sur le côté distal de la lésion et une région intermédiaire entre ledit côté proximal et ledit côté distal ayant un diamètre plus grand que ledit diamètre relativement grand, ledit système comprenant :
un stent-prothèse (100) ayant une première extrémité (104), une seconde extrémité (102) et une section médiane (106), ladite première extrémité (101) dudit stent-prothèse ayant un diamètre agrandi qui est égal ou légèrement supérieur au diamètre de la région médiane du vaisseau sanguin, ladite section médiane (106) dudit stent-prothèse (100) ayant un diamètre agrandi qui est approximativement égal audit diamètre relativement petit dudit vaisseau sanguin,
dans lequel ladite seconde extrémité (102) dudit premier stent-prothèse (100) a un diamètre agrandi qui est égal ou légèrement supérieur au diamètre relativement grand du vaisseau sanguin, de façon telle que lorsque ledit système est installé, ladite première extrémité (104) dudit stent-prothèse (100) peut entrer en contact avec la région intermédiaire du vaisseau sanguin et ladite seconde extrémité (102) dudit premier stent-prothèse (100) peut entrer en contact avec le côté proximal de la lésion dans le vaisseau sanguin, et
un second stent-prothèse (200) ayant une première extrémité (202) et une seconde extrémité (204), ladite première extrémité (202) dudit second stent-prothèse (200) ayant un diamètre agrandi égal ou légèrement plus grand que ledit diamètre agrandi de ladite section médiane (106) dudit premier stent-prothèse (100) et ladite seconde extrémité (204) dudit second stent-prothèse (200) ayant un diamètre agrandi qui est égal ou légèrement supérieur au relativement petit diamètre du vaisseau sanguin, de façon telle que lorsque ledit système est installé, ladite première extrémité (202) dudit second stent-prothèse (200) peut entrer en contact avec ladite section médiane (106) dudit premier stent-prothèse (100), et ladite seconde extrémité (204) dudit second stent-prothèse (200) peut entrer en contact avec le vaisseau sanguin sur le côté distal de la lésion.

2. Système selon la revendication 1 dans lequel ladite première extrémité (104) et/ou la seconde extrémité (102) dudit stent-prothèse (100) est évasée.

3. Système selon la revendication 1 ou 2 comprenant en outre :
un troisième stent-prothèse (500) ayant une première extrémité (502) et une seconde extrémité (504), ladite première extrémité dudit troisième stent-prothèse (500) ayant un diamètre agrandi égal ou légèrement supérieur audit diamètre agrandi de ladite section médiane (206) du second stent-prothèse (200) de sorte que lorsque ledit système est installé, ladite première extrémité (502) dudit troisième stent-prothèse (500) peut entrer en contact avec ladite section médiane (206) dudit second stent-prothèse (200).

4. Système selon la revendication 3 dans lequel ladite première extrémité (502) dudit troisième stent-prothèse (500) est évasée.

5. Système selon l'une quelconque des revendications 1 à 3 dans lequel ladite première extrémité (104) et/ou ladite seconde extrémité (102) dudit premier stent-prothèse (100) est évasée.

6. Système selon l'une quelconque des revendications 1 à 5 dans lequel au moins ladite section médiane (106) dudit premier stent-prothèse (100) est renforcée par un matériau flexible (105).

7. Système selon la revendication 6 dans lequel ledit matériau flexible (105) comprend du téréphtalate de polyéthylène, du nylon, du polytétrafluarcéthylène, de la polyoléfine, du polyamide, du polycarbonate, un uréthane de polycarbonate ou un fil métallique.

8. Système selon la revendication 6 dans lequel ledit matériau flexible (105) est sélectionné parmi les sutures, les tricots, les tissages, les tresses, les fils et les stents.

9. Système selon l'une quelconque des revendications 1 à 8 dans lequel ladite première extrémité (202) dudit second stent-prothèse (200) est couplée audit premier stent-prothèse (100) par l'un des éléments suivants : sutures, fils, adhésif et soudures.

10. Système modulaire de stent-prothèse selon l'une quelconque des revendications 1 à 9 dans lequel ladite section médiane dudit premier stent-prothèse (100) définit un premier passage la traversant, et ladite section médiane (206) dudit second stent-prothèse (200) définit un second passage la traversant, ladite première extrémité (202) dudit second stent-prothèse (200) étant couplée audit premier stent-prothèse (100) de façon telle que ledit premier passage est substantiellement contigu audit second passage.

11. Système selon la revendication 10 dans lequel ladite première extrémité (202) dudit second stent-prothèse (200) est couplée à ladite section médiane (106) dudit premier stent-prothèse (100).
